# EUROPEAN PATENT SPECIFICATION

(11) **EP 1 603 598 B1**
(45) Date of publication and mention of the grant of the patent: **08.10.2008**
(21) Application number: 04721547.0
(22) Date of filing: 18.03.2004
(51) Int. Cl.: A61K 49/08, A61K 51/06, A61P 35/00

(54) **RADIOLABELED CONJUGATES BASED ON SUBSTANCE P AND THE USES THEREOF**
AUF SUBSTANZ P BASIERENDE RADIOMARKIERTE KONJUGATE UND DEREN GEBRAUCH
CONJUGES RADIOMARQUES BASES SUR LA SUBSTANCE P ET LEUR UTILISATION

(30) Priority: 19.03.2003 EP 03006061
(43) Date of publication of application: 14.12.2005
(73) Proprietor: Universitätsspital Basel, 4031 Basel (CH); Universität Bern, 3012 Bern (CH)
(72) Inventor: MERLO, Adrian, CH-4312 Magden (CH); MÄCKE, Helmut, 79539 Lörrach (DE); REUBI, Jean-Claude, CH-3084 Wabern (CH); GOOD, Stephan, CH-4153 Reinach (CH)
(74) Representative: Schaad, Balass, Menzl & Partner AG
(86) International application number: PCT/EP2004/050329
(87) International publication number: WO 2004/082722

(56) References cited:
- EP-A- 1 283 216
- WO-A-01/02021
- WO-A-02/24235
- WO-A-20/04035623
- FICHNA J. ET AL: "Synthesis of target specific radiolabeled peptides for diagnostic imaging" BIOCONJUGATE CHEM, vol. 14, 2003, pages 3-17, XP001172817
- NIKULA, T. K. ET AL: "Improved method for labelling DOTA-analogues with 213Bi, 225Ac and lanthanides" ABSTRACTS OF PAPERS 225 ACS NATIONAL MEETING, 23 March 2003 (2003-03-23), - 27 March 2003 (2003-03-27) page Nucl-067, XP008023978
- LAZNICEK M ET AL: "Octreotide and octreotate derivatives radiolabeled with yttrium: Pharmacokinetics in rats." CANCER BIOTHERAPY AND RADIOPHARMACEUTICALS, vol. 17, no. 5, 2002, pages 527-533, XP008023912 ISSN: 1084-9785
- HEPPELER A ET AL: "RECEPTOR TARGETING FOR TUMOR LOCALISATION AND THERAPY WITH RADIOPETIDES" CURRENT MEDICINAL CHEMISTRY, BENTHAM SCIENCE PUBLISHERS BV, BE, vol. 7, no. 9, 2000, pages 971-994, XP000982225 ISSN: 0929-8673

## Description

The instant invention relates to a pharmaceutical composition comprising a pharmaceutical carrier and an effective amount of a substance P analogue conjugate with 1-(1,3-carboxypropyl)-4,7,10-(carboxymethyl)-1,4,7,10-tetraazacyclododecane (chelator DOTAGA), 1-(1,2-carboxyethyl)-4,7,10-(carboxymethyl)-1,4,7,10-tetraazacyclododecane (chelator DOTASA) or 1,4,7,10-tetraazaacyclo-dodecane-1,4,7,10-tetra-acetic acid (chelator DOTA), that are labelled with suitable radionuclides; the use thereof for targeting and treatment of brain tumors or the use of corresponding labelled substance P conjugates for targeting and treatment of brain tumors; substance P analogue conjugates with DOTAGA, DOTASA or DOTA, that are labelled with suitable radionuclides; and substance P analogue conjugates with DOTAGA, DOTASA or DOTA and their t-butylesters.

Substance P is a naturally occurring 11-amino acid neuropeptide and belongs to the family of bioactive neuropeptides known as the tachykinins [Payan (1989) Ann. Rev.Med. 40, 341]. The amino acid sequence of this undecapeptide was identified as H-Arg1¹-Pro²-Lys³-Pro⁴-Gln⁵-Gln⁶-Phe⁷-Phe⁸-Gly⁹-Leu¹⁰-Met¹¹-NH₂. In WO 92/18536 is described a method for detecting and localizing tissues, having neurokinine 1 (NK1) receptors, such as tumors with NK1 receptors in the body of a warm-blooded living being, by administration of a composition, comprising a labelled small peptide, e.g. substance P and analogue derivatives having a selective affinity to neurokinine 1 receptors, and by then assaying that living being. Furthermore the invention relates to a method for the therapeutic treatment of malignant tumors, e.g. glioma, small cell lung cancer and others.

J. Fichna et al. summarize in Bioconjugate Chem. 2003, pages 3 to 17 synthesis of target-specific radio-labelled peptides for diagnostic imaging and possibilities for application as radiopharmaceuticals. Various peptides and conjugates with various chelating molecules are mentioned, but the combination of substance P and its analogues with DOTA or DOTAGA is not described and there is no hint to treatment of brain tumors with this radio-labelled conjugates.

The prochelator (ester) and chelator (add) DOTA 1,4,7,10-tetraazaacyclo-dodecane-1,4,7-tris(acetic acid-t-butyl ester)-10-acetic acid is described in Chem.Eur.J.1999,5, No. 7, Seiten 1974-1981.

Successful studies on loco-regional regulatory peptide receptor targeting with a diffusible somatostatin analogue ¹¹¹In/⁹⁰Y-labeled-DOTA⁰-D-Phe¹-Tyr³-octreotide (DOTATOC), a radio-labelled samostatine based conjugate, showed for the first time in vivo in human brain tumors patients that DOTATOC has high affinity binding to SSTR-2 (somatostatin subtype 2 receptor) in the low nanomolar range, which has been reported by A. Merlo et al (1999) in Clin. Cancer Res. 5, 1025-1033. Furthermore S. Hofer et al report in Swiss Med. Weekly 2001, 131: 640-644 the effect of diffusible brachytherapy (dBT) using the locally injected stable radioconjugate ⁸⁰Y-labeled-DOTA⁰-D-Phe¹-Tyr³-octreotide (DOTATOC) for managing symptomatic low grade gliomas.

EP-A- 1 283 216 relates to somatostatin analogues comprising among other alternatives a DOTA-based chelator and the use of the somatostatin analogues in the treatment of diseases characterized by an overexpression of somatostatin receptors. Further, M. Laznicek et al have in Cancer Biotherapy and Radiopharmaceuticals, Vol. 17, No. 5, 2002, 527 - 533 reported on the distribution profiles and elimination pathways of octreotate derivatives radiolabeled with yttrium.

WO 01/02 021 relates to a radiolabelled tachy kinin peptide analogue and its use for mammalian in vivo tachy kinin peptide receptor imaging.

In WO 02/24235 is described the preparation of 1-(1-carboxy-3-carbo-tert.-butoxypropyl)-4,7,10-(carbo-tert-butoxy-methyl)-1,4,7,10-tetraazacyclododecane (prochelator DOTAGA), which is used to prepare conjugates of effector molecules and 1-(1,3-carboxy-propyl)-4,7,10-(carboxy-methyl)-1,4,7,10-tetraazacyclododecane (chelator DOTAGA). As effector molecules are proposed peptides such as octreotide or CCK, which are coupled to the N-terminus of these bioactive peptides. Conjugates with substance P have also been mentioned, but have not prepared and therefore not exemplified. Said conjugates may be labelled with radio-nuclides for both diagnostic (¹¹¹In, ^{67/68}Ga) and internal radio-therapeutic applications (⁹⁰Y, ¹⁷⁷Lu). The publication is focused on octreotide and octreotate conjugates such as DOTATOC, which provide a better labelling yield and a high stability. The treatment of brain tumors , e.g. gliomas, is not mentioned.

It was surprisingly found that the NK-1 receptor is expressed in brain tumors and can be applied for binding radiolabelled conjugates based on substance P and analogues thereof with chalators DOTAGA, DOTASA or DOTA and that said conjugates are much more effective than DOTATOC, radiolabelled substance P, substance P and analogues and saporin, and other small radiolabelled peptides with or without chelating agent in targeting and treatment of tumors, especially brain tumors, e.g. gliomas. It was also surprisingly found that metal complex formation is achieved within short time and that the complexes possess a higher chemical stability, thus avoiding contamination of tissue or body fluids with free radio-nuclides. Substance P based radio-labelled conjugates show an unexpected high serum half-life time sufficient for application as internal radio-diagnostics and radio-therapeutics. Serum stability of conjugates on the basis of substance P analogues is even increased without decreasing the binding affinity to NK1 receptor to an undesired extent. In some cases the binding affinity is even improved when using substance P analogues. It was also found that methionin in the 11-position is oxidation sensitive (radiolysis) after labelling (forming a sulfoxide) and that said oxidation can be suppressed in replacing methionin by methioninsulfone, whereby the binding affinity can substantially be maintained or even improved with further amino acid replacements in the substance P sequence. It was also surprisingly found that substance P based radio-labelled conjugates are capable to diffuse after administration and infiltrate tumor cell nests or satellite lesions, so that they can be detected and treated.

A first object of the invention is a radio-nuclide labelled conjugate of substance P and a chelator molecule, having the abbreviation Chelator-R-Arg¹-Pro²-Lys³-Pro⁴-Gln⁵-Gln⁶-Phe⁷-Phe⁸-Gly⁹-Leu¹⁰-Met¹¹-NH₂ and comprising compounds of formula I 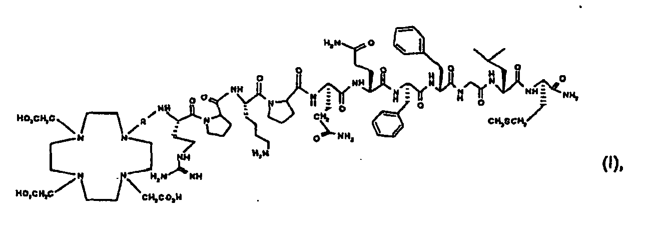 wherein
R is -CH₂-C(O)-, -C(CO₂H)CH₂CH₂C(O)- or -C(CO₂H)CH₂-C(O)-,
modified by the subsequent modification a) in the amino acid sequence of substance P:
a) replacement of Met¹¹ by -NH-CH(CH₂-CH₂-SO₂-CH₃)-C(O)-(Met(O₂)¹¹), -NH-CH(CH₂CH₂-SO-CH₃)-C(O)-(Met(O)¹¹), or -NH-CH[CH(CH₃)CH₂CH₃)-C(O)-(Ile¹¹), and optionally at least one of the subsequent modifications b) to g):
b) replacement of Leu¹⁰ by -NH-CH[CH(CH₃)CH₂CH₃)-C(O)- (Ile¹⁰),
c) replacement of Gly⁹ by -N(CH₃)-CH₂C(O)-(Sar⁹),
d) replacement of Phe⁷ or Phe⁸ or both Phe⁷ and Phe⁸ by residue of formulae 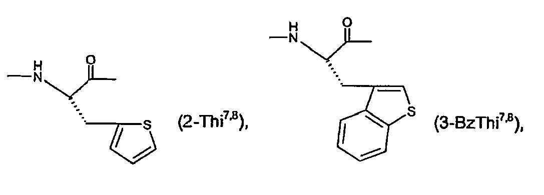 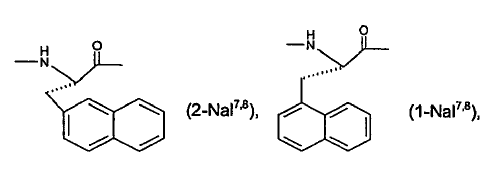 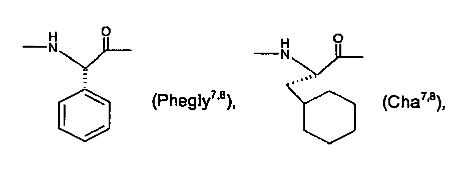
e) replacement of Lys³ by residue of formulae 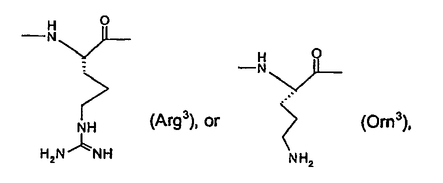
f) truncation of 1 to 5 amino acids of the sequence Arg¹-Pro²-Lys³-Pro⁴-Gln⁵, or
g) replacement of 1 to 5 amino acids of the sequence Arg¹-Pro²-Lys³-Pro⁴-Gln⁵ by -N(CH₃)-CH₂-C(O)- (Sar),
and wherein the conjugate is labelled with a radio-nuclide selected from the group consisting of Actinium-225, Bismut-212, Bismut-213, Lead-203, Copper-64, Copper-67, Gallium-66, Gallium-67, Gallium-68, Lutetium-177, Indium-111, Indium-113, Yttrium-86 and Yttrium-90, Dyprosium162, Dysprosium 165, Dysprosium 167, Holmium-166, Praseodymium-142, Praseodymium-143, Promethium-149, and Terblum-149, and their use as active ingredient in radio-pharmaceutical or radio-diagnostic formulations for targeting or treating brain tumors, especially gilomas.

When R in formula I attached to the tetraazamacrocyclic residue is -CH₂-C(O)-, it is abbreviated as chelator DOTA; when R in formula I attached to the tetraazamacrocyclic residue is -C(CO₂H)CH₂CH₂-C(O)-, it is abbreviated as chelator DOTAGA; and when R in formula I attached to the tetraazacyclic residue is -C(CO₂H)CH₂-C(O)-, it is abbreviated as chelator DOTASA. When the carboxylic groups in the chelator moieties are esterified, for example with t-butanol, then the residues are named prochelator. The prochelators can be used for convenient coupling to peptides during solid phase synthesis.

Labelling in the context of this invention means binding the radionuclide to the carboxylic groups and nitrogen atoms of the chelator residue, whereby anions in the radionuclide salts are replaced in accordance with the valence of the metal ion, which are substantially metal(III) or metal(II) ions.

The abbreviations used for modifying amino acids are explained in the following: Met(O) is mothionine-sulfoxide; Met(O₂) is methionine-sulfone; Ile is isoleucine; Sar is sarcosine; 2-Thi is 3-(2-thienyl)-alanine; 3-BzThl is 3-(3-benzothienyl)-alanine; 2-Nal is 3-(2-naphthyl)-alanine; 1-Nal is 3-(1-naphthyl)-alanine; Cha is cyclohoxyl-alanine; Arg is arginine; Orn is ornithine; and Phegly is phenylglycin.

In a preferred embodiment of the invention, the residue R in formula I means -CH₂-C(O)-In a further preferred embodiment, the amino acid sequence in formula I corresponds to formulae
a) Arg-Pro-Lys-Pro-Gln-Gln-Phe-Phe-Gly-Leu-Met(O₂)-NH₂,
b) Arg-Pro-Lys-Pro-Gln-Gln-Phe-Phe-Sar-Leu-Met(O₂)-NH₂,
c) Arg-Pro-Lys-Pro-Gln-Gln-Phe-Thi-Gly-Leu-Met(O₂)-NH₂,
d) Arg-Pro-Lys-Pro-Gln-Gln-Thi-Phe-Gly-Leu-Met(O₂)-NH₂,
e) Arg-Pro-Lys-Pro-Gln-Gln-Phe-Thi-Sar-Leu-Met(O₂)-NH₂,
f) Arg-Pro-Lys-Pro-Gln-Gln-Thi-Phe-Sar-Leu-Met(O₂)NH₂
g) Arg-Pro-Lys-Pro-Gln-Gln-Thi-Thi-Sar-Leu-Met(O₂)-NH₂,
h) Arg-Pro-Lys-Pro-Gln-Gln-Thi-Thi-Gly-Leu-Met(O₂)-NH₂.

In another preferred embodiment, the compounds of formula I comprise in the 11-position of the natural substance P sequence a methioninsulfone residue of formula -NH-CH(CH₂CH₂-SO₂CH₃)-C(O)- instead of a methionin residue. It was found that the methionin residue is prone to oxidation/is easier oxidized in air and especially during the labelling process with radionuclides, which leads to inconsistent final products, Inconsistency can be avoided when using methioninsulfone instead of methionin.

Another preferred substance P analogue conjugate of formula I is one in which the glycin residue in position 9 of the natural substance P sequence is replaced by a sarcosin residue of formula -N(CH₃)-CH₂-C(O)-.

Another preferred substance P analogue conjugate of formula I is one in which the phenylalanine residue in the 7- or 8-position or in both positions of the natural substance P sequence is replaced by a 3-(2-thienyl)-alanine residue of formula

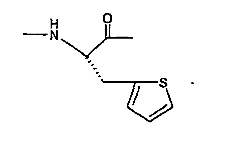

Another preferred substance P analogue conjugate of formula I is one in which the phenylalanine residue in the 8-position of the natural substance P sequence is replaced by a 3-(2-thienyl)-alanine and the glycin residue in position 9 is replaced by a sarcosine residue.

Another preferred substance P analogue conjugate of formula I is one in which the methionin residue in the 11-position of the natural substance P sequence is replaced by a methioninsulfone residue, and the phenylalanine residue in the 8-position of the natural substance P sequence is replaced by a 3-(2-thienyl)-alanine residue, or the glycin residue in position 9 is replaced by a sarcosine residue.

Substance P analogue conjugates of formula I, which are modified in the 8-position as described before, show a much better serum stability or a lower peptidase sensibility, respectively. Substance P analogue conjugates of formula I, which are modified in the 7-, 8-, 9- and/or 11-position as described before, show also a satisfying to high binding affinity to NK-1 receptor compared to natural substance P, as it can be detected with autoradiography in measuring the IC₅₀ values, which may be from 0.1 to 50, preferably from 0.5 to 20 and more preferably from 0.7 to 10.

In a more preferred embodiment the amino acid sequence in formula I corresponds to formulae
a) Arg-Pro-Lys-Pro-Gln-Gln-Phe-Phe-Gly-Leu-Met(O₂)-NH₂,
b) Arg-Pro-Lys-Pro-Gln-Gln-Phe-Phe-Sar-Leu-Met(O₂)-NH₂,
c) Arg-Pro-Lys-Pro-Gln-Gln-Phe-Thi-Gly-Leu-Met(O₂)-NH₂,
d) Arg-Pro-Lys-Pro-Gln-Gln-Phe-Thi-Sar-Leu-Met(O₂)-NH₂.

In a particularly preferred embodiment the amino acid sequence in formula I corresponds to formulae
a) Arg-Pro-Lys-Pro-Gln-Gln-Phe-Phe-Sar-Leu-Met(O₂)-NH₂, or
b) Arg-Pro-Lys-Pro-Gln-Gln-Phe-Thi-Gly-Leu-Met(O₂)-NH₂,
since these compounds show an outstanding oxidation and serum stability as well as a very high binding affinity, which is comparable or even higher than with natural substance P sequence.

The conjugates of formula I and the preferred radioconjugates mentioned before, having either chelators DOTAGA, DOTASA or DOTA bound to the amino terminus are useful for diagnostic investigations using conventional scintigraphy with Indium-111 labelled substance P or analogues or using positron emission tomography with the tracers Gallium-68, Gallium-66, Yttrium-86 or Copper-64. The diagnostic peptide vector can either be applied loco-regionally or systemically.

For therapeutic treatment the conjugates of formula I and the analogue conjugates mentioned before with either chelators DOTAGA, DOTASA or DOTA can be labelled for example with alpha-emitting isotopes, e.g. Bismuth-213, Actinium-225, and the beta-emitters Yttrium-90 and Lutetium-177.

Another aspect of the invention provides a composition for targeting brain tumors, localizing or treating brain tumors and the satellite lesions thereof in a host afflicted with brain tumors, e.g. gliomas, comprising at least one compound of formula I. The composition is especially useful in the detection and therapeutic treatment of brain tumors and satellite lesions thereof.

An object of the invention is also a therapeutic or diagnostic composition for targeting brain tumors, localizing or treating brain tumors and the satellite lesions thereof in a mammal comprising administering to a mammal in need of such therapy, an effective amount of a radio-nuclide labelled substance P conjugate of formula I.

Another object of the invention is a composition for delivering a radio-nuclide labelled substance P conjugate of formula I to a host, comprising administering to a host a radio-nuclide labelled substance P conjugate of formula I to diagnose or to treat human brain tumors.

A further object of the invention is the use of a radio-nuclide labelled substance P conjugate of formula I for the manufacture of a medicament useful for the detection and therapeutic treatment of brain tumors and satellite lesions thereof in a mammal, such as a human; and a radio-nuclide labelled substance P conjugate of formula I for use in medical therapy.

The object of the invention are conjugates of substance P analogues and a chelator molecule, whereby substance P conjugate having the abbreviation Chelator-R-Arg¹-Pro²-Lys³-Pro⁴-Gln⁵-Gln⁶-Phe⁷-Phe⁸-Gly⁹-Leu¹⁰-Met¹¹-NH² and comprising compounds of formula I. 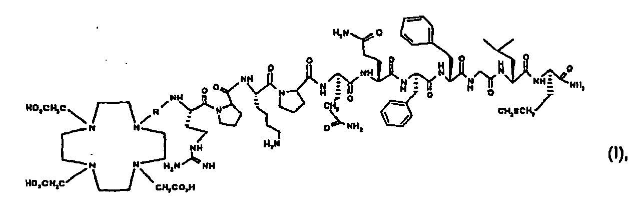 wherein
R is -CH₂-C(O)-, -C(CO₂H)CH₂CH₂-C(O)- or -C(CO₂H)CH₂-C(O)-, is modified by the subsequent modification a) in the amino acid sequence of substance P:
a) replacement of Met" by -NH-CH(CH₂CH₂-SO₂CH₃)-C(O)- (Met(O₂)¹¹), -NH-CH(CH₂CH₂-SO-CH₃)-C(O)- (Met(O)¹¹), or -NH-CH[CH(CH₃)CH₂CH₃)-C(O)- (Ile¹¹),
   and optionally at least one of the subsequent modifications b) to g):
b) replacement of Leu¹⁰ by -NH-CH[CH(CH₃)CH₂CH₃)-C(O)-(Ile¹⁰),
c) replacement of Gly⁹ by -N(CH₃)-CH₂C(O)- (Sar⁹),
d) replacement of Phe⁷ or Phe⁸ or both Phe⁷ and Phe⁸ by residue of formulae 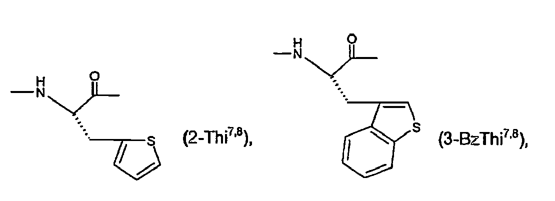 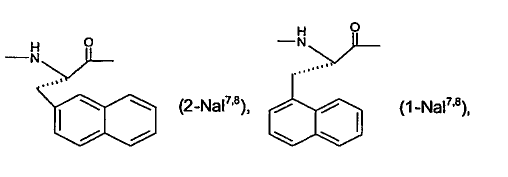 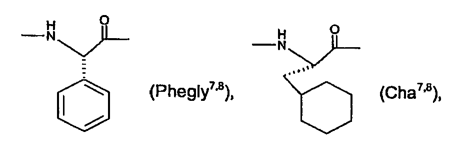
e) replacement of Lys³ by residue of formulae 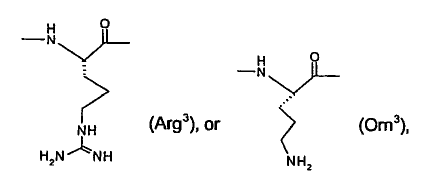
f) truncation of 1 to 5 amino acids of the sequence Arg¹-Pro²-Lys³-Pro⁴-Gln⁵, or
g) replacement of 1 to 5 amino acids of the sequence Arg¹-Pro²-Lys³-Pro⁴-Gln⁵ by -N(CH₃)-CH₂C(O)- (Sar),
and wherein the conjugates are unlabelled or labelled with a radionuclide selected from the group consisting of Actinium-225, Bismut-212, Bismut-213, Lead-203, Copper-64, Copper-67, Gallium-66, Gallium-67, Gallium-68, Lutetium-177, Indium-111, Indium-113, Yttrium-86 and Yttrium-90, Dyprosium162, Dysprosium 165, Dysprosium 167, Holmium-166, Praseodymium-142, Praseodymium-143, Promethium-149, and Terbium-149; including preferred embodiments of the analogues as mentioned before.

Still a further object of the invention is a composition comprising (a) at least one pharmaceutical carrier and (b) at least one conjugate of an analogue of substance P and a chelator molecule, whereby substance P conjugate having the abbreviation Chelator-R-Arg¹-Pro²-Lys³-Pro⁴-Gln⁵-Gln⁶-Phe⁷-Phe⁸-Gly⁹-Leu¹⁰-Met¹¹-NH₂ and comprising compounds of formula I 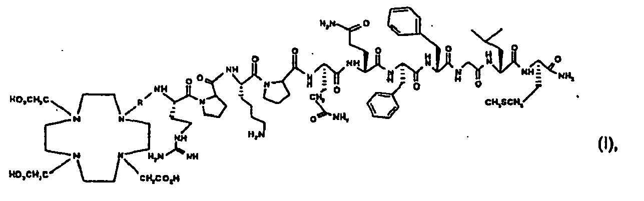 wherein
R is -CH₂-C(O)-, -C(CO₂H)CH₂CH₂-C(O)- or -C(CO₂H)CH₂C(O)-, is modified by the subsequent modification a) in the amino acid sequence of substance P:
a) replacement of Met¹¹ by -NH-CH(CH₂CH₂-SO₂-CH₃-C(O)- (Met(O₂)¹¹), -NH-CH(CH₂CH₂-SO-CH₃)-C(O)-(Met(O)¹¹), or-NH-CH[CH(CH₃)CH₂CH₃)-C(O)-(Ile¹¹), and optionally at least one of the subsequent modifications b to g):
b) replacement of Leu¹⁰ by -NH-CH[CH(CH₃)CH₂CH₃)-C(O)- (Ile¹⁰),
c) replacement of Gly⁹ by -N(CH₃)-CH₂C(O)- (Sar⁹),
d) replacement of Phe⁷ or Phe⁸ or both Phe⁷ and Phe⁸ by residue of formulae 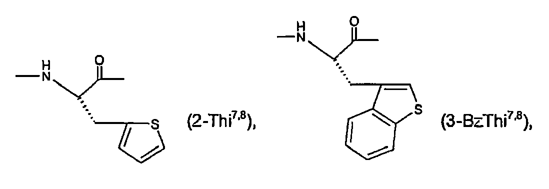 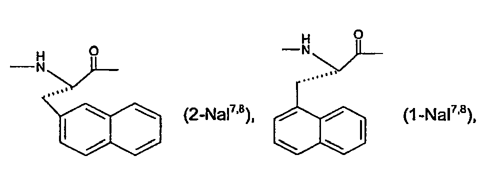 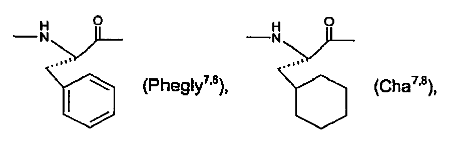
e) replacement of Lys³ by residue of formulae 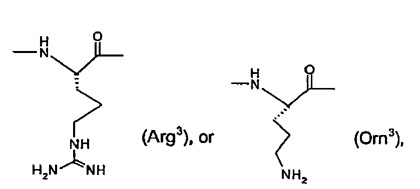
f) truncation of 1 to 5 amino acids of the sequence Arg¹-Pro²-Lys³-Pro⁴-Gln⁵, or
g) replacement of 1 to 5 amino acids of the sequence Arg¹-Pro²-Lys³-pro⁴-Gln⁵ by -N(CH₃)-CH₂₋C(O)-(Sar),
and wherein the conjugates are labelled with a radionuclide selected from the group consisting of Actinium-225, Bismut-212, Bismut-213, Lead-203, Copper-64, Copper-67, Gallium-66, Gallium-67, Gallium-68, Lutetium-177, indium-111, indium-113, Yttrium-66 and Yttrium-90, Dyprosium162, Dysprosium 165, Dysprosium 167, Holmium-166, Praseodymium-142, Praseodymium-143, Promethium-149, and Terbium-149; including preferred embodiments as mentioned before.

The invention includes the pharmaceutically acceptable salts and complexes of the radio-nuclide labelled conjugates of formula I, and of the unlabelled conjugates of formula I. Salts may be formed from inorganic or organic acids. Examples of suitable inorganic acids are hydrochloric acid, hydrofluoric acid, hydrobromic acid, hydroiodic acid, sulfuric acid and boric acid. Examples of suitable organic acids are acetic acid, trifluoroacetic acid, formic acid, oxalic acid, malonic acid, succinic acid, tartaric acid, maleic acid, fumaric acid, methanesulfonic acid, trifluoromethanesulfonic acid, benzoic acid, glycolic acid, lactic acid, citric acid and mandelic acid. Salts may be formed from inorganic or organic bases, such as alkaline metal hydroxides, and hydroxides from ammonia, hydroxyethylamine, hydrazine, methylamine, ethylamine, trimethylamine, triethylamine, ethylenediamine, hydroxyethylamine, morpholine, piperazine and guanidine.

The radionuclide labelled conjugates may be used in pharmaceutical formulations as powders (micronized particles), granules, suspensions or solutions, or they may be combined together with other pharmaceutically acceptable ingredients in admixing the components and optionally finely divide them, and then filling capsules, composed for example from hard or soft gelatine, preparing transdermal patches, compressing tablets, pills or troches, or suspend or dissolve them in carriers for solutions, suspensions, elixirs and syrups. Coatings may be applied after compression to form pills.

Pharmaceutically acceptable ingredients are well known for the various types of formulation and may be for example binders such as natural or synthetic polymers, solvents, excipients, lubricants, surfactants, sweetening and flavouring agents, coating materials, preservatives, dyes, thickeners, adjuvants, antimicrobial agents, stabilizers, osmo-regulators, and other carriers for the various formulation types.

The radionuclide labelled substance P conjugates of formula I for targeting and treatment of brain tumors may be administered by every known route and may be selected from the group consisting of the intravenous route, the intraarterial route, the intra-cutaneus route, the subcutaneous route, the oral route, the buccal route, the intramuscular route, the anal route, the transdermal route, the intradermal route, the intrathecal route and the like.

In one preferred embodiment, the pharmaceutical carrier is a liquid and the pharmaceutical composition is in the form of a suspension, emulsion and preferably a solution.

Examples for liquid carriers are water, alcohols such as ethanol, glycerol, propylene glycol, liquid polyethylene glycols, triacetin and oils, which may be used alone or in combination of at least two components.

The amount of a radionuclide labelled conjugate in the formulation substantially depends on type of formulation and desired dosages during administration time periods. The amount may be from 0.01 to 40 percent by weight, preferably from 0.1 to 25 percent by weight, and more preferably from 0.5 to 15 percent by weight, referred to the pharmaceutical composition.

Liquid pharmaceutical compositions, which are sterile solutions or suspensions can be utilized for intramuscuslar, intrathecal, intratracheal, epidural, intraperitoneal or subcutaneaus injections. Sterile solutions can also be administered intravenously. The conjugatess may be prepared as a sterile solid composition which may be dissolved or suspended (diffused) at the time of administration using sterile water, saline, or other appropriate sterile injectable medium.

Particularly preferred is the local injection of diffusible peptide vectors to be used according to the invention for loco-regional application. The radiopharmaceutical is injected either into a stereotactically implanted ventricular catheter or into a capsule from which a catheter leads to the tumor center or into the resection cavity (also called port-a-cath-device). A number of different, commercially available capsules and catheters can be used for this purpose.

In non-resected main tumor masses, the tip of the catheter has to be centered into the putative midportion of the tumor which can normally be achieved using a 3-dimensional stereotactic planning system. It is important to wait at least one day, preferably a few days, between catheter insertion and the first application in order to control the problem of back flow along the outside of the catheter away from the target site into the subarachnoid, subdural, epidural or subcutaneous space. This phenomenon can further be reduced by lowering the elevated intracranial pressure using osmodiuretics and high dose dexamethasone prior to application of the radiopharmaceutical. In solid non-resected cases, a slow infusion technique is used taking advantage of an infusion pump that allows continuous infusion of a volume of 5-10ml over a time period of 1-2 hours. This technique is distinct from the so called "convection enhanced delivery" of macromolecules into brain parenchyma that uses even slower infusion velocities to allow some penetration of large molecules that do not diffuse due to their size. The conjugates used according to the invention are virtually small drugs (1-2 kDaltons) and display highly diffusive properties. Diffusion across large areas of a tumor, even across the corpus callosum to the other hemisphere, has been repeatedly observed within 30 minutes following a bolus injection of 2-3 ml of the radiopharmaceutical used according to the invention.

The radionuclide labelled substance P conjugates of formula I can be used alone or in combination with other pharmaceutically active substances. In the case of combinations with other pharmaceutically active compounds, a fixed combination of two or more components (e.g. kit of parts) are prepared as already known to a person of skill in the art, and the said conjugates and any other active compound are administered at an interval that allows common, additional or preferably synergistic effect for brain tumor and/or treatment, e.g. targeting and treatment of gliomas.

In any treatment regimen, the pharmaceutical compositions of the radionuclide labelled substance P conjugates of formula I may be administered to a patient either singly or in a cocktail containing two or more targeted toxins, other therapeutic agents or compositions, including for example immunosuppressive agents, tolerance-inducing agents, potentiators and side-effect relieving agents. Particularly preferred side-effect relieving agents are those useful in suppressing allergic reactions of a host. Preferred immunosuppressive agents include prednisone, DECADRON (Merck, Sharp and Dohme, West Point, Pa.), cyclophosphamide, cyclosporine, 6-mercaptopurine, methotrexate, azathioprine and gamma globulin or their combination. Preferred potentiators include monensin, ammonium chloride, perhexiline, verapamil amantadine and chloroquine. All of these agents are administered in generally-accepted efficacious dose ranges.

Optimal dosages to be administered may be determined by those skilled in the art, and will vary with the particular target and compound in use, the strength of the preparation, the mode of administration, and the advancement of the disease condition. Additional factors depending on the subject being treated, including subject age, weight, gender, diet and time of administration, will result in a need to adjust dosages for this special purpose. Administration of the conjugates and optionally additional pharmaceutical agents may be effected continuously or intermittently.

In the treatment, an appropriate dosage level will generally be 0.001 to 50 mg/kg patient body weight per day which can be administered in single or multiple doses. Preferably, the dosage level will be 0.005 to 25 mg/kg per day, more preferably 0.01 to 10 mg/kg per day, and even most preferably 0.05 to 1 mg/kg per day.

The substance P conjugates, of formula I and the radionuclide labelled conjugates of substance P of formula I are prepared in known manner following standard solid phase synthesis (SPPS) in a common side chain protected form as usually being the preferred version of synthesis.

Solid phase peptide synthesis (SPPS) may be carried out on an Applied Biosystems Model 431 A or 432 A Peptide synthesizer using for example Fmoc (9-fluorenemethoxycarbonyl) strategy. The general principles and methods followed are well known in the art. For a more precise description it is referred to " Fluorenemethoxycarbonyl-polyamide solid phase synthesis: General Synthesis and Development"- Chapter 3 in "Solid peptide Synthesis: A practical approach" by E. Atherton and R.C. Sheppard, Information Press Ltd. Oxford , U.K. (1989).

The obtained undecapeptides (substance P or analogues) are coupled for example with the prochelators DOTAGA(^{t}Bu)₄, DOTASA(^{t}Bu)₄ or DOTA(^{t}Bu)₃. After cleavage from the resin and optional deprotection the products may be purified by preparative HPLC to a purity higher than 99,5%.

The obtained substance P conjugates of formula I may be labelled with radio-nuclides in known manner optionally with or after deprotection to form the carboxylic acid groups bound to the tetraazacyclic residue. Labelling may be carried out in aqueous solution in contacting the deprotected conjugate with radionuclide salts from inorganic or organic acids such as halogenides (chlorides, bromides, iodides), sulfates, nitrates, sulfonates such as phenyl- or toluyl sulfonate, formiates, acetates, benzoate or oxalates.

The invention is further illustrated in the following examples, where synthesis and application of substance P conjugates, of formula I and radionuclide labelled conjugates of substance P of formula I is described in more detail.

### A) Preparation of substance P conjugates and substance P analogue conjugates (However, substance P conjugates which do not fall under the general formula (I) do not from part of the invention)

### a) General preparation procedure for peptide synthesis

As already mentioned earlier, peptides are synthesized on a solid phase using fmoc-strategy (fmoc = 9-Fluorenylmethoxycarbonyl). To achieve N-terminal amides, Rink amide MBHA resin is used. The synthesis is performed on a semiautomatic peptide synthesizer from RINK Engineering, Bubendorf, Switzerland. This synthesizer is equipped with one or up to 10 reation vessels. The aminoacids are purchased from Novabiochem AG, Läufelfingen, Switzerland. All other reagents are purchased from Fluka Chemie GmbH, Buchs, Switzerland.

In general, 1 equivalent of Rink-amide MBHA resin with a theoretical loading of 0.7 mmole/g resin is given to the reactor. Dimethylformamide (DMF) is added to the reactor and is shaken for 15 minutes to allow swelling of the resin. After removing the solvent, again a portion of DMF is added during 2 minutes. To the pre-swelled resin, a solution of 20% piperidine in DMF is added within 5 minutes to remove the fmoc-protection from the resin. This step is repeated once within 5 minutes and then again but within 12 minutes. After this procedure, the solid phase is washed twice for 0.5 minutes with DMF and once again for 1 min with DMF. The piperidine solutions and the DMF solutions of the last three washings are collected and filled with ethanol (EtOH) to 500 ml. From this solution an aliquot is taken to determine the amount of removed fmoc-protecting groups spectrophotometrically. Therefore, the absorption of the solution at 300 nm is determined and the original fmoc-amount is calculated.

Before coupling the fmoc-amino acid derivative to the solid phase, the resin is washed twice for 2 minutes with N-methyl-pyrolidinone (NMP). Meanwhile, 3 equivalents of the fmoc-protected aminoacid derivative together with 3.3 equivalents of 1-hydroxybenzotriazole and 3.3 equivalents of N,N'-diisopropylcarbodiimide are dissolved in NMP and incubated for 45 minutes. Then, this coupling solution is added to the solid phase and the pH is adjusted to a value of 7-8 by adding about 5 equivalents of N,N-diisopropylethylamine (DIPEA). The reaction is incubated for 90 minutes under gentle shaking. After the reaction solution is removed, the solid phase is washed twice with DMF for 1 minute. The reaction is controlled performing a Kaiser-test: About 10 beads of the solid phase are washed 5 times with ethanol. 20 g phenol in 10 ml ethanol are mixed with 1 ml of a solution of 1 ml 0.01 M KCN in 49 ml pyridine. 50 µl of this mixture are added to the beads followed by 10 µl of a solution of 500 g ninhydrine in 10 ml ethanol. The beads are heated for 10 minutes to 95 °C. Blue beads indicate remainning free amino functions.

After attachment of the first amino acid, the following fmoc-amino acids are coupled in a similar manner but with a slightly different time program. The synthesizer is therefore programmed as described in the following table:

| Step | Operation | Repetitions | Time (min) | Collect waste |
|---|---|---|---|---|
| 1 | DMF | 2 | 2 | |
| 2 | Piperidin/DMF | 2 | 5 | Yes |
| 3 | Piperidin/DMF | 1 | 9 | Yes |
| 4 | DMF | 2 | 0.5 | Yes |
| 5 | DMF | 1 | 1 | Yes |
| 6 | NMP | 2 | 2 | |
| 7 | Reaction | 1 | 90 | |
| 8 | DMF | 2 | 1 | |

All amino acids are used as N-terminal fmoc-protected derivatives. Lysine is used with tert.-butoxycarbonyl (Boc) protecting group on the side chain amino function. Arginine is used with 2,2,4,6,7-pentamethyldihydro-benzofuran-5-sulfonyl (Pbf) protection of the side chain. If the peptide synthesis is performed in a single reactor. Kaiser test is performed after coupling of each amino acid. If Kaiser test indicates remaining free amino functions, the coupling of the amino acid is repeated.

After building of the whole desired peptide sequence, the solid phase is washed 5 times with isopropanol followed by 5 times washing with diethyl ether, each for 1 minute. For 5 min, a constant air stream is drying the solid phase.

### b) Coupling with prochelator DOTAGA(^{t}Bu)₄ and DOTA(^{t}Bu)₃

The prochelator DOTAGA(^{t}Bu)₄ is synthesized as described in WO 02/24235 (H.R. Mäcke et al). DOTA(^{t}Bu)₃ is purchased at Macrocyclics Inc., Dallas, USA. Prior coupling the prochelator, the N-terminal fmoc-protection is removed from the resin bound peptides. Therefore, it is swelled for 15 minutes in DMF, washed again for 2 minutes with DMF and treated then twice with a solution of 20% piperidin in DMF for 5 minutes. Then, the solid phase is treated another 12 minutes with this solution and washed twice for 0.5 minutes with DMF and for another minute again with DMF. The solution from the piperidin treatments and the following DMF washings are collected to determine the content of cleaved fmoc groups. 2 equivalents of Prochelator and 1.2 equivalents of O-(7-azabenzotriatol-1-yl)-N,N,N'N'-tetramethyluronium hexafluorophosphate (HATU) are dissolved in DMF and incubated for 45 minute. After this, 2 equivalents of DIPEA are added to the solution. This reaction solution is added then to the solid phase and after 2 minutes, the pH of the reaction is controlled and, if needed, adjusted to a value between 7 and 8 by adding some more DIPEA. The reaction mixture is shaked over night. After removing the solution, the solid phase is washed twice with DMF for 2 minutes, 5 times with isopropanol and 5 times with diethyl ether for 1 minute. It is dried then in an air stream for 10 minutes.

### c) Deprotection, cleavage and purification

The solid phase is given to a syringe equipped with a frit. A solution of 91 % trifluoroacetic acid (TFA), 5% thioanisole, 3% water and 1 % triisopropylsilane is added and the syringe is agitated for 1 h. The solution is then poured to a polypropylene tube. To this solution is added a mixture of 50% diisopropylether and 50% petrolether. For 30 min the tube is cooled at -20°C. The precipitation is collected by centrifugation of the tube at 1006 g for 5 minutes and the solvent is decanted. Meanwhile, the solid phase in the syringe is treated again with the upper deprotection solution for 2 h. The deprotection solution is added to the already achieved precipitation and the solid is diluted with this. After another 3 h of deprotection, the crude product is precipitated again, cooled, centrifuged and separated from the solvent by decantation. The crude product is kept then at a vacuum (<100 mbar) over night to remove the remaining solvents.
The crude product is dissolved in a mixture of acetonitrile : water = 1 : 1 and purified by preparative HPLC on a Macherey-Nagel VP Nucleosil 100-5C18, 21x250 mm. The preformed eluent gradient is the following: A = acetonitrile, B = 0.1% trifluoroacetic acid (TFA) in water; flow = 15 ml/min; 0 min, 15% A; 25 min, 50% A; 26 min, 100% A; 28 min, 100% A; 30 min, 15% A. The main peak is collected and evaporated to dryness. It is analyzed by analytical HPLC and mass spectroscopy (MALDI-TOF-MS). A Macherey-Nagel CC250/4 Nucleosil 120-3C18 column is used for analytical separation, using the follwing eluent gradient: A = acetonitrile, B = 0.1% TFA in water; flow = 0.75 ml/min; 0 min, 90% A; 20 min, 40% A; 23 min, 0% A; 24 min, 0% A; 27 min, 90% A.

The following conjugates are prepared:
DOTAGA means a residue 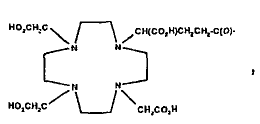 and
DOTA means a residue

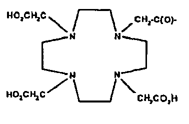

### Example A1:

DOTAGA-Arg¹-Pro²-Lys³-Pro⁴-Gln⁵-Gln⁶-Phe⁷-Phe⁸-Gly⁹-Leu¹⁰-Met¹¹-NH₂
DOTAGA-Substance P: C₈₂H₁₂₈N₂₂O₂₂S, calculated (m/z): 1806.1, found 1807.2; R_{f}: 29.8.

### Example A2:

DOTA-Arg¹-Pro²-Lys³-Pro⁴-Gln⁵-Gln⁶-Phe⁷-Phe⁸-Gly⁹-Leu¹⁰-Met(O₂)¹¹-NH₂
DOTA-[Met(O₂)¹¹]-Substance P: C₇₉H₁₂₆N₂₂O₂₂S, calculated (m/z): 1766.9, found 1766.2; R_{f}: 26.0.

### Example A3:

DOTA-Arg¹-Pro²-Lys³-Pro⁴-Gln⁵-Gln⁶-Phe⁷-Phe⁸-Sar⁹-Leu¹⁰-Met¹¹-NH₂
DOTA-[Sar⁹]-Substance P: C₈₀H₁₂₈N₂₂O₂₂S, calculated (m/z): 1748.9, found 1748.5; R_{f}: 28.03.

### Example A4:

DOTA-Arg¹-Pro²-Lys³-Pro⁴-Gln⁵-Gln⁶-Phe⁷-Thi⁸-Gly⁹-Leu¹⁰-Met¹¹-NH₂
DOTA-[Thi⁸]-Substance P: C₇₇H₁₂₄N₂₂O₂₀S₂. calculated (m/z): 1740.9, found 1740.7; R_{f}: 27.87.

### Example A5:

DOTA-Arg¹-Pro²-Lys³-Pro⁴-Gln⁵-Gln⁶-Thi⁷-Phe⁸-Gly⁹-Leu¹⁰-Met¹¹-NH₂
DOTA-[Thi⁸]-Substance P: C₇₇H₁₂₄N₂₂O₂₀S₂, calculated (m/z): 1740.9, found 1740.6; R_{f}: 27.66.

### Example A6:

DOTA-Arg¹-Pro²-Lys³-Pro⁴-Gln⁵-Gln⁶-Phe⁷-Phe⁸-Sar⁹-Leu¹⁰-Met(O₂)¹¹-NH₂
DOTA-[Sar⁹, Met(O₂)¹¹]-Substance P: C₈₀H₁₂₈N₂₂O₂₂S, calculated (m/z): 1780.9, found 1780.1; R_{f}: 25.34.

### Example A7:

DOTA-Arg¹-Pro²-Lys³-Pro⁴-Gln⁵-Gln⁶-Phe⁷-Thi⁸-Gly⁹-Leu¹⁰-Met(O₂)¹¹-NH₂
DOTA-[Thi⁸, Met(O₂)¹¹]-Substance P: C₇₇H₁₂₄N₂₂O₂₂S₂, calculated (m/z): 1772.9, found 1772.7; R_{f}: 25.34.

### Example A8:

DOTA-Arg¹-Pro²-Lys³-Pro⁴-Gln⁵-Gln⁶-Phe⁷-Thi⁸-Sar⁹-Leu¹⁰-Met¹¹-NH₂
DOTA-[Thi⁸, Sar⁹]-Substance P: C₇₈H₁₂₆N₂₂O₂₀S₂, calculated (m/z): 1754.9, found 1754.9; R_{f}: 26.70.

### Example A9:

DOTA-Arg¹-Pro²-Lys³-Pro⁴-Gln⁵-Gln⁶- Thi⁷- Thi⁸-Gly⁹-Leu¹⁰-Met¹¹-NH₂
DOTA-[Thi⁷, Thi⁸]-Substance P: C₇₅H₁₂₂N₂₂O₂₀S₃, calculated (m/z): 1746.8, found 1746.4; R_{f}: 27.29.

### B) Preparation of radionuclide labelled conjugates

### General procedure

For ⁹⁰Y-/¹¹¹In-DOTAGA-Substance P: To a sterile 30 µg aliquot of the chelator-peptide conjugate in water is added an aqueous 30 mCi ⁹⁰YCl₃-solution or 0.5 mCi ¹¹¹InCl₃-solution and filled up to 500 µl with sterile 0.4 M NaOAc-Buffer (pH 5). This solution is heated for 30 minutes to 95°C and cooled to room temperature for 15 minutes. The solution is diluted to 1 ml with physiological NaCl-solution. A sample of it is controlled on the radio-HPLC for determining the content of non-bound radionuclide. Using ⁹⁰Y-DOTAGA-Substance P, it was found that a minor amount of the oxidation product ⁹⁰Y-DOTAGA-[Met(O)¹¹]-Substance P is formed. Analytical HPLC is carried out as described under example A(c).

The following radionuclide labelled conjugates are prepared:

### Example B1:

¹¹¹In-DOTAGA-Arg¹-Pro²-Lys³-Pro⁴-Gln⁵-Gln⁶-Phe⁷-Phe⁸-Gly⁹-Leu¹⁰-Met¹¹-NH₂
¹¹¹In-DOTAGA-Substance P:
C₈₂H₁₂₅InN₂₂O₂₂S calculated (m/z): 1916,81, found: 1917.6 (M+H)⁺, R_{f}: 29.80.

### Example B2:

⁹⁰Y-DOTAGA-Arg¹-Pro²-Lys³-Pro⁴-Gln⁵-Gln⁶-Phe⁷-Phe⁸-Gly⁹-Leu¹⁰-Met¹¹-NH₂
⁹⁰Y-DOTAGA-Substance P

### Example B3:

¹¹¹In-DOTA-Arg¹-Pro²-Lys³-Pro⁴-Gln⁵-Gln⁶-Phe⁷-Phe⁸-Gly⁹-Leu¹⁰-Met(O₂)¹¹-NH₂
¹¹¹In-DOTA-[Met(O₂)¹¹]-Substance P

### Example B4:

¹¹¹In-DOTA-Arg¹-Pro²-Lys³-Pro⁴-Gln⁵-Gln⁶-Phe⁷-Phe⁸-Sar⁹-Leu¹⁰-Met¹¹-NH₂
¹¹¹In-DOTA-[Sar⁹]-Substance P

### Example B5:

¹¹¹In-DOTA-Arg¹-Pro²-Lys³-Pro⁴-Gln⁵-Gln⁶-Phe⁷-Thi⁸-Gly⁹-Leu¹⁰-Met¹¹-NH₂
¹¹¹In-DOTA-[Thi⁸]-Substance P

### Example B6:

¹¹¹In-DOTA-Arg¹-Pro²-Lys³-Pro⁴-Gln⁵-Gln⁶-Thi⁷-Phe⁸-Gly⁹-Leu¹⁰-Met¹¹-NH₂
¹¹¹In-DOTA-[Thi⁸]-Substance P

### Example B7:

¹¹¹In-DOTA-Arg¹-Pro²-Lys³-Pro⁴-Gln⁵-Gln⁶-Phe⁷-Phe⁸-Sar⁹-Leu¹⁰-Met(O₂)¹¹-NH₂
¹¹¹In-DOTA-[Sar⁹, Met(O₂)¹¹]-Substance P

### Example B8:

¹¹¹In-DOTA-Arg¹-Pro²-Lys³-Pro⁴-Gln⁵-Gln⁶-Phe⁷-Thi⁸-Gly⁹-Leu¹⁰-Met(O₂)¹¹-NH₂
¹¹¹In-DOTA-[Thi⁸, Met(O₂)¹¹]-Substance P

### Example B9:

¹¹¹In-DOTA-Arg¹-Pro²-Lys³-Pro⁴-Gln⁵-Gln⁶-Phe⁷-Thi⁸-Sar⁹-Leu¹⁰-Met¹¹-NH₂
¹¹¹In-DOTA-[Thi⁸, Sar⁹]-Substance P

### Example B10:

¹¹¹In-DOTA-Arg¹-Pro²-Lys³-Pro⁴-Gln⁵-Gln⁶-Thi⁷-Thi⁸-Gly⁹-Leu¹⁰-Met¹¹-NH₂
¹¹¹In-DOTA-[Thi⁷, Thi⁸]-Substance P

### Example B11: Labelling protocol

The general procedure as described under B) is repeated at 50 °C and binding of ⁹⁰YCl₃ is detected after defined reaction times. From the result in the following table can be seen that the reaction can be considered as complete after about 15 minutes.

| Reaction time (min) | Labelling yield (%) |
|---|---|
| 2 | 73.276 |
| 3 | 80.975 |
| 5 | 89.996 |
| 10 | 97.666 |
| 15 | 99.423 |
| 20 | 99.567 |
| 30 | 99.660 |
| 60 | 99.709 |

### C) Application examples

### Example C1: General observations

The radionuclide substance P conjugates and analogues thereof being undeca-peptides are prepared following standard solid phase synthesis (SPPS) in a common side-chain protectted form and the obtained peptide coupled to the prochelator DOTAGA(^{t}Bu)₄. After cleavage and following deprotection DOTAGA-Substance P and analogues are purified by preparative HPLC to a purity higher than 95%. The obtained substance P and analogue conjugates are sterilized and thereafter labeled with a radionuclide salt as mentioned above, e.g. ¹¹¹InCl₃ (18 MBq per application) for imaging and ⁹⁰YCl₃ (37 MBq/µg DOTAGA-Substance P) using for example 0.4M sodium acetate buffer adjusted to pH 5.0 containing ascorbic acid (2g/10ml) and 2,5-dihydroxy benzoic acid (370 mg/19 ml).

The labelling can also be performed with ²¹³Bi, a very promising alpha emitter available from a Ac-225/Bi-213 generator. The labelling yields are in the same range as for Y-90 and In-111 (>95%), but Bi-213 has a short half-life time of only 47 min.

During the labelling process a second peak is identified on the HPLC chromatogram which is identified as ⁹⁰Y/¹¹¹In-DOTAGA-[Met(O)¹¹]-Substance P as a product of radiolysis during the labelling process. Unfortunately this derivative showes a binding affinity to NK1 receptors by a factor 11 lower than the non-oxidized substance. Even by an addition of a large amount of an reducing agent, e.g. ascorbic acid to the buffer, the unwanted oxidation can not be suppressed completely. On the other side the synthesized derivative ⁹⁰Y/¹¹¹In-DOTAGA-or DOTA-[Sar⁹ Met(O₂)¹¹]-Substance P show an extremely higher affinity than the sulfoxide-derivatives (less than a factor of 2 lower than the native sequence). Thus it underlines that Met(O₂) at position 11 of the peptide sequence can be introduced without a significant loss of affinity and eliminates the eventually upcoming oxidation problems.

Further stability test by in vitro experiments incubating ⁹⁰Y-DOTAGA-Substance P in different CSF (cerbospinal fluid) aspirates from patients are executed. A significant difference in degradation of the radiopeptide correlating with the "quality" of the CSF probe can be demonstrated. If the aspirate is colourless and of a clear aspect (does not contain blood), the labelled conjugate is stable for several hours, while in aspirates with a visible blood serum contamination, the substance is completely degraded within 5h.

This enzymatic degradation is also of high impact in patients, as seen in different urine samples from 0 to 24 h p.i. and CSF aspirates 1 week p.i. All samples show the same two radioactive metabolites as in the in vitro experiment with serum contaminated CSF probes. The quantification of the radioactivity in the urine also indicates a very fast wash-out of the applied ⁹⁰Y bound on metabolites of DOTAGA-Substance P.

### Example C2: Stability test (radionuclide transfer to competetive chelator DTPA)

⁹⁰Y-DOTAGA-substance P is dissolved in water at pH 7 and admixed with a 10⁵ excess of diethylenetriamine penta-acetic acid (DTPA). The amount of intact radionuclide is determined after certain time periods. The results, indicating the high stability, are given in the following table.

| Time (h) | Intact part (%) |
|---|---|
| 0 | 100 |
| 73 | 99.361 |
| 168 | 98.483 |
| 234 | 98.030 |
| 405 | 97.185 |

### Example C3: Metabolic blood serum stability

¹¹¹Y labelled compounds as mentioned in the table are contacted with blood serum and the amount of intact parts is determined after certain time periods. The results, indicating the higher stability of the modified substance P conjugate, are given in the following table.

| Time (h) | ¹¹¹In-DOTAGA-[Thi⁸, Met(O₂)¹¹]-Substance P ¹¹¹In-DOTA-[Thi⁸, Met(O₂)¹¹]-Substance P (Intact parts±SEM) | ¹¹¹In-DOTAGA-Substance P ¹¹¹In-DOTA-Substance P (Intact parts±SEM) |
|---|---|---|
| 0 | 1.00±0.2 | 1.00±0.2 |
| 1 | 0.93±0.01 | 0.79±0.01 |
| 2 | 0.82±0.01 | 0.54±0.07 |
| 4 | 0.69±0.02 | 0.29±0.14 |
| 6 | 0.56±0.07 | 0.15±0.10 |
| 8 | 0.47±0.04 | 0.08±0.08 |
| 24 | 0.16±0.10 | 0.00±0.00 |

### Example C4: Binding affinity to NK-1 receptors

In vitro autoradiography is performed with tumor tissue expressing NK1 receptors. The used radioligand is ¹²⁵I-Bolton-Hunter-SP. Displacement experiments are performed on successive sections with the various compounds listed in the Table given in concentrations ranging from 0.1 nM to 1'000 nM. IC₅₀ values are calculated from 10 radiolabeled distinct substance P analogues and compared to natural SP. The results are given in the following table.

| | |
|---|---|
| Substance | IC50±SEM (mean value) |
| Substance P | 2.7±0.22 |
| ¹¹¹In-DOTAGA-Substance P | 1.1 |
| ¹¹¹In-DOTAGA-[MET(O)¹¹]-Substance P | 9.8±1.00 |
| ¹¹¹In-DOTA-[MET(O₂)¹¹]-Substance P | 3.55±0.45 |
| ¹¹¹In-DOTA-[Sar⁹]-Substance P | 3.20±0.30 |
| ¹¹¹In-DOTA-[Thi⁸]-Substance P | 7.30±2.00 |
| ¹¹¹In-DOTA-[Thi⁷]-Substance P | 9.40±1.60 |
| ¹¹¹In-DOTA-[Sar⁹, MET(O₂)¹¹]-Substance P | 2.00±0.00 |
| ¹¹¹In-DOTA-[Thi⁸, MET(O₂)¹¹]-Substance P | 0.78±0.03 |
| ¹¹¹In-DOTA-[Thi⁸, Sar⁹]-Substance P | 3.40±0.40 |
| ¹¹¹In-DOTA-[Thi⁷, Thi⁸]-Substance P | 7.70±0.70 |

### Example C5: Receptor autoradiography in primary brain tumors comparing the somatostatin and the neurokinin-1 (NK-1) targeting system

An immunohistochemical study of representative cases of primary gliomas conducted, show that the distinction between tumor infiltrated brain tissue and normal brain is, in many instances, virtually impossible because of the background expression of somatostatin recaptors on normal brain tissue. Despite this observation, In-111 DOTATOC scintigrams show confinement of the injected radiopharmakon to the tumor compartment as visualized on MRI. This might best be explained by the chaotic structure of the extracellular tissue architecture within the tumor and the infiltrated brain as compared to non-infiltrated brain tissue. Bakay has shown in his publication (Brain 1970, 93, 693-698) that the extracellular space can make up 20-40% of a given volume of brain tumor tissue as compared to normal brain which contains about 5% of extracellular space. Physically speaking, a tumor utilizes the available energy to proliferate and infiltrate, and not to maximize the order of the molecular architectture as normal brain tissue does (law of entropia). It has been described the consistent and high expression of neurokinin-1 receptors in glioblastomas, but also in low- and intermediate grade gliomas. Accordingly a comparative autoradiographic study between the somatostatin/DOTATOC and the substance P/NK-1 system has been conducted in order to define the best targeting compounds for locoregional therapy in brain tumors. With one single exception of an ependymoma, the expression of NK-1 receptors is at least equivalent or higher in most gliomas of grades II-IV. The results teach that the substance P/NK-1 system can replace the somatostatin/DOTATOC system easily yielding superior responses due to a clearly more favourable tumor to brain background ratio.

Example C6: Pilot Study with ⁹⁰Y-DOTAGA-Substance P treatment In the following substance P pilot study, 4 cases with biopsy alone followed by ⁹⁰Y-DOTAGA-Substance P (treatment) are included; all cases with deep or eloquently located tumors where open surgery is not deemed advisable as a first treatment option, and 5 cases with open gross total resection and subsequent radionuclide conjugate brachytherapy. As a third group which can serve as a control, 5 advanced cases that had been treated by the so called standard regimens with virtually no therapeutic option left anymore were included. Since this is a non-controlled pilot study, it had to be relied on the results of the only randomized large glioma trial executed and published more than 20 years ago (Walker et al, 1980, N Engl J Med). In cases with biopsy alone, average survival is around 9 weeks without further treatment. In the 4 cases of this pilot study, all patients survived at least 2 months and up to 9 months, and in a special case even show a remarkable partial response. In the resected 5 cases followed by internal radionuclide conjugate brachytherapy, overall survival appeared to be longer than expected which is in the range between 14 and 26 months. According to the only large randomized trial of 1970s which serves as historical control, resection alone would have led to an expected survival of about 4 months, and resection plus an external beam radiotherapy to a mean survival of about 9.5 months. Quite remarkably, all survival numbers are located on the right of these expected values on the time axis which can be taken as strong indication that the conjugate Y-90DOTAGA substance P is certainly more efficient than a pure water solution.

This interpretation of the data is further supported by an impressive partial response (case W.M.) which is obtained in a non-resectable left-sided thalamic glioblastoma, using ⁹⁰Y-DOTAGA-Substance P monotherapy and where a volume reduction of over 80% of the glioblastoma was observed. A key question in local glioma therapy is whether one can achieve to reach infiltrating tumor cell nests or satellite lesion. In an illuminating case with ¹¹¹In-DOTAGA-Substance P, diffusibility is shown in a 4 cm distant satellite lesion that becomes visible following intracavitary injection into there section cavity. Despite good labelling, the satellite lesion could not be controlled with Y-90 substance P. This case proves that even if the target can be labelled, a radionuclide with a narrower range would have been much more likely to eradicate this lesion. In contrast the presently alpha-emitters (µm range) and narrow range beta-emitters (e.g. Lu-177, 1-2 mm) should prove superior in the treatment of invasive tumor cell clusters. The presently used dissipative Y-90 (range 3-6 mm) is more suitable for bulky lesions.

## Claims

1. A conjugate of a substance P analogue and a chelator molecule, whereby substance P conjugate having the abbreviation Chelator-R-Arg¹-Pro²-Lys³-Pro⁴-Gln⁵-Gln⁶-Phe⁷-Phe⁸-Gly⁹-Leu¹⁰-Met¹¹-NH₂ and comprising compounds of formula I 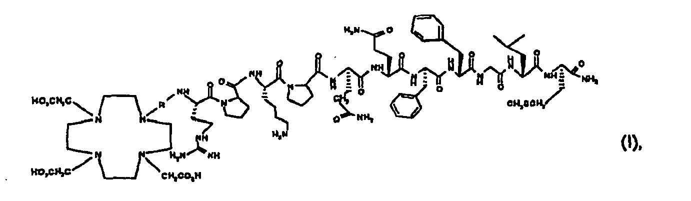 wherein
R is -CH₂-C(O)-, -C(CO₂H)CH₂CH₂-C(O)- or -C(CO₂H)CH₂-C(O)-, is modified by the subsequent modification a) in the amino acid sequence of substance P:
a) replacement of Met¹¹ by -NH-CH(CH₂CH₂-SO₂-CH₃)-C(O)- (Met(O₂)¹¹), -NH-CH(CH₂CH₂-SO-CH₃)-C(O)- (Met(O)¹¹), or -MH-CH[CH(CH₂)CH₂CH₃)-C(O)- (Ile¹¹), and optionally at least one of the subsequent modifications b) to g):
b) replacement of Leu¹⁰ by -NH-CH[CH(CH₂)CH₂CH₃)-C(O)- (Ile¹⁰),
c) replacement of Gly⁹ by -N(CH₃)-CH₂-C(O)- (Sar⁹),
d) replacement of Phe⁷ or Phe⁸ or both Phe⁷ and Phe⁸ by residue of formulae 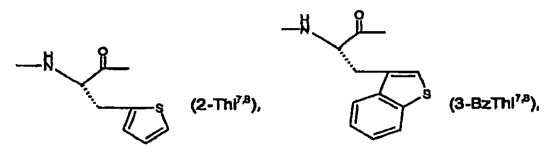 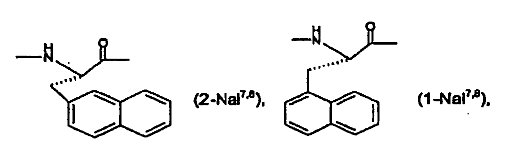 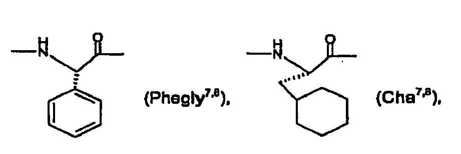
e) replacement of Lys³ by residue of formulae 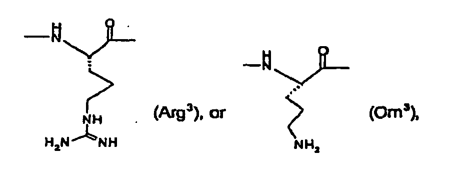
f) truncation of 1 to 5 amino acids of the sequence Arg¹-Pro²-Lys³-Pro⁴-Gln⁶, or
g) replacement of 1 to 5 amino acids of the sequence Arg¹-Pro²-Lys³-Pro⁴-Gln⁵ by -N(CH₃)-CH₂-C(O)- (Sar),
and wherein the conjugates are unlabelled or labelled with a radio-nuclide selected from the group consisting of Actinium-225, Bismut-212, Bismut-213, Lead-203, Copper-64, Copper-67, Gallium-66, Gallium-67, Gallium-68, Lutetium-177, Indium-111, Indium-113, Yttrium-86 and Yttrium-90, Dyprosium162, Dysprosium 165, Dysprosium 167, Holmium-166, Praseodymium-142, Praseodymium-143, Promethium-149, and Terbium-149.

2. The conjugate of claim 1 wherein in the amino acid sequence of substance P Met¹¹ is replaced by -NH-CH (CH₂CH₂-SO₂-CH₃) -C (O) - (Met (O₂)¹¹) .

3. A composition comprising (a) at least one pharmaceutical carrier and (b) at least one of the labelled conjugates according to claim 1 or 2.

4. The labelled conjugate of claim 1 or 2 for use in medical therapy.

5. The labelled conjugate of claim 1 or 2 for targeting or treating brain tumors, especially gliomas.

6. The labelled conjugate of claim 1 or 2 for locoregional application thereof by injection either into a sterestactically implanted ventricular catheter or into a capsule from which a catheter leads to the tumor center or into the resection cavity of a host.

7. Use of a labelled conjugate according to claim 1 or 2 for the manufacture of radiopharmaceutical or radio-diagnostic formulations for targeting or treating brain tumors, especially gliomas.

## Patentansprüche

1. Ein Konjugat eines Substanz P-Analogons und eines Chelator-Moleküls, wobei das Substanz P-Konjugat, welches die Abkürzung Chelator-R-Arg¹-Pro²-Lys³-Pro⁴-Gln⁵-Gln⁶-Phe⁷-Phe⁸-Gly⁹-Leu¹⁰-Met¹¹-NH₂ aufweist und Verbindungen der Formel I umfasst, 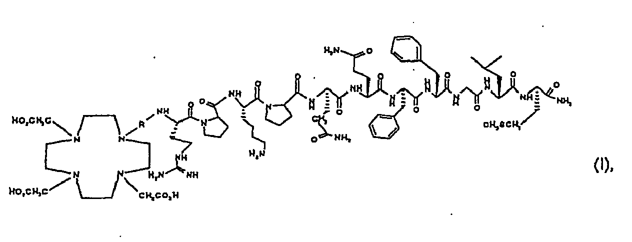 in der R -CH₂-C(O)-, -C(CO₂H)CH₂CH₂-C(O)- oder -C(CO₂H)CH₂-C(O)- ist, mit der folgenden Modifikation a) in der Aminosäuresequenz von Substanz P modifiziert ist:
a) Austausch von Met¹¹ durch -NH-CH(CH₂CH₂-SO₂-CH₃)-C(O)-(Met (O₂)¹¹), -NH-CH(CH₂CH₂-SO-CH₃) -C(O) - (Met (O)¹¹) oder -NH-CH[CH(CH₃)CH₂CH₃)-C(O)- (Ile¹¹)
und wahlweise mit mindestens einer der folgenden Modifikationen b) bis g):
b) Austausch von Leu¹⁰ durch -NH-CH[CH(CH₃)CH₂CH₃)-C(O)-(Ile¹⁰),
c) Austausch von Gly⁹ durch -N(CH₃)-CH₂-C(O)- (Sar⁹),
d) Austausch von Phe⁷ oder Phe⁸ oder sowohl von Phe⁷ als auch von Phe⁸ durch einen Rest der Formeln 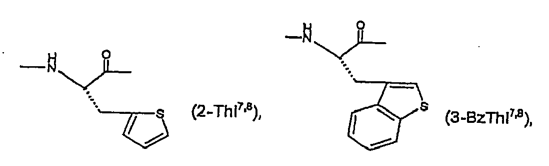 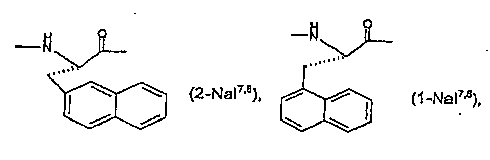 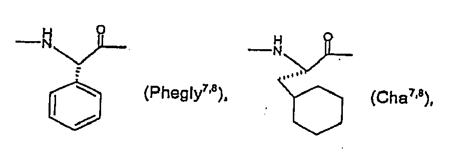
e) Austausch von Lys³ durch einen Rest der Formeln 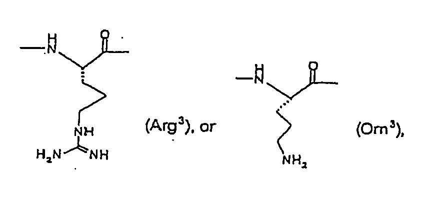
f) Kürzung von 1 bis 5 Aminosäuren der Sequenz Arg¹-Pro²-Lys³-Pro⁴-Gln⁵ oder
g) Austausch von 1 bis 5 Aminosäuren der Sequenz Arg¹-Pro²-Lys³-Pro⁴-Gln⁵ durch -N(CH₃)-CH₂-C(O)- (Sar),
und wobei die Konjugate unmarkiert oder mit einem Radionuklid ausgewählt aus der Gruppe bestehend aus Actinium-225, Bismut-212, Bismut-213, Blei-203, Kupfer-64, Kupfer-67, Gallium-66, Gallium-67, Gallium-68, Lutetium-177, Indium-111, Indium-113, Yttrium-86 und Yttrium-90, Dysprosium 162, Dysprosium 165, Dysprosium 167, Holmium-166, Praseodymium-142, Praseodymium-143, Promethium-149 und Terbium-149 markiert sind.

2. Das Konjugat nach Anspruch 1, wobei in der Aminosäuresequenz von Substanz P Met¹¹ durch -NH-CH(CH₂CH₂-SO₂-CH₃)-C(O)- (Met(O₂)¹¹) ersetzt ist.

3. Eine Zusammensetzung enthaltend (a) wenigstens einen pharmazeutischen Träger und (b) wenigstens eines der markierten Konjugate gemäss Anspruch 1 oder 2.

4. Das markierte Konjugat nach Anspruch 1 oder 2 zur Verwendung in einer medizinischen Therapie.

5. Das markierte Konjugat nach Anspruch 1 oder 2 zur gezielten Markierung oder zur Behandlung von Hirntumoren, insbesondere Gliomen.

6. Das markierte Konjugat nach Anspruch 1 oder 2 zur lokoregionalen Verabreichung desselben mittels Injektion entweder in einen stereotaktisch implantierten ventrikulären Katheter oder in eine Kapsel, von welcher aus ein Katheter zum Tumorzentrum und/oder in die Resektionshöhle eines Empfängers führt.

7. Verwendung eines markierten Konjugats gemäss Anspruch 1 oder 2 zur Herstellung von radiopharmazeutischen oder radiodiagnostischen Formulierungen zur gezielten Markierung oder zur Behandlung von Hirntumoren, insbesondere Gliomen.

## Revendications

1. Conjugué d'un analogue de substance P et d'une molécule de chélateur, **caractérisé en ce que** le conjugué de substance P ayant l'abréviation Chélateur-R-Arg¹-Pro²-Lys³-Pro⁴-Gln⁵-Gln⁶-Phe⁷-Phe⁸-Gly⁹-Leu¹⁰-Met¹¹-NH₂ et comprenant des composés de formule I 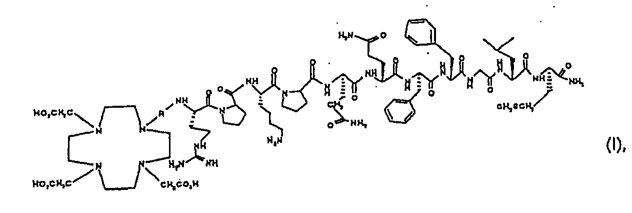 dans laquelle
R est -(CH₂)-C(O)-, -C(CO₂H)CH₂CH₂-C(O)- ou -C(CO₂H)CH₂-C (O) -,
est modifié par la modification suivante a) dans la séquence d'acides aminés de la substance P :
a) remplacement de Met¹¹ par -NH-CH (CH₂CH₂-SO₂-CH₃)-C (O)-(Met (O₂)¹¹), -NH-CH(CH₂CH₂-SO-CH₃)-C(O)-(Met(O)¹¹), ou -NH-CH(CH(CH₃)CH₂CH₃)-C(O)-(Ile¹¹),
et éventuellement au moins une des modifications suivantes b) à g) :
b) remplacement de Leu¹⁰ par NH-CH (CH (CH₃) CH₂CH₃) -C (O)-(Ile¹⁰),
c) remplacement de Gly⁹ par -N(CH₃)-CH₂-C(O)-(Sar⁹),
d) remplacement de Phe⁷ ou Phe⁸ ou de Phe⁷ et Phe⁸ par un résidu de formule 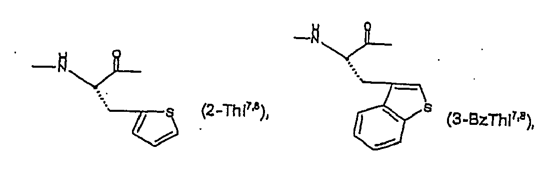 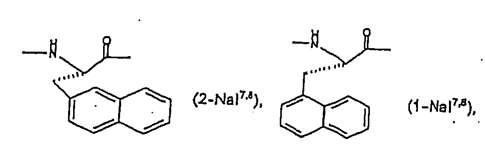 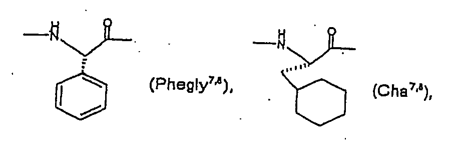
e) remplacement de Lys³ par un résidu de formule 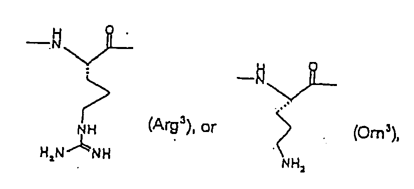
f) troncature de 1 à 5 acides aminés de la séquence Arg¹-Pro²-Lys³-Pro⁴-Gln⁵, ou
g) remplacement de 1 à 5 acides aminés de la séquence Arg¹-Pro²-Lys³-Pro⁴-Gln⁵ par -N(CH₃)-CH₂-C(O)-(Sar),
et **caractérisé en ce que** les conjugués sont non marqués ou marqués avec un radionucléide choisi dans le groupe constitué des actinium 225, bismuth 212, bismuth 213, plomb 203, cuivre 64, cuivre 67, gallium 66, gallium 67, gallium 68, lutétium 177, indium 111, indium 113, yttrium 86 et yttrium 90, dysprosium 162, dysprosium 165, dysprosium 167, holmium 168, praséodyme 142, praséodyme 143, prométhéum 149 et terbium 149.

2. Conjugué de la revendication 1 **caractérisé en ce que** dans la séquence d'acides aminés de la substance P Met¹¹ est remplacé par -NH-CH(CH₂CH₂-SO₂-CH₃)-C(O)-(Met (O₂)¹¹) .

3. Composition comprenant (a) au moins un véhicule pharmaceutique et (b) au moins un des conjugués marqués selon la revendication 1 ou 2.

4. Conjugué marqué de la revendication 1 ou 2 pour une utilisation en thérapie médicale.

5. Conjugué marqué de la revendication 1 ou 2 pour cibler ou traiter des tumeurs du cerveau, en particulier des gliomes.

6. Conjugué marqué de la revendication 1 ou 2 pour l'application locorégionale par injection dans un cathéter ventriculaire implanté par stéréotaxie ou dans une capsule depuis laquelle un cathéter conduit au centre de la tumeur ou dans la cavité de résection d'un hôte.

7. Utilisation d'un conjugué marqué selon la revendication 1 ou 2 pour la fabrication de formulations radiopharmaceutiques ou radiodiagnostiques pour cibler ou traiter des tumeurs du cerveau, en particulier des gliomes.
